# EUROPÄISCHE PATENTSCHRIFT

(11) **EP 0 895 782 B1**
(45) Veröffentlichungstag und Bekanntmachung des Hinweises auf die Patenterteilung: **01.12.2004**
(21) Anmeldenummer: 98113643.5
(22) Anmeldetag: 22.07.1998
(51) Int. Cl.: A61K 47/12, A61K 31/475, A61K 9/20

(54) **"Gegen Racemisierung stabilisierte pharmazeutische Zubereitungen von Cilansetron"**
Pharmaceutical compositions of Cilansetron stabilized against racemisation
Compositions pharmaceutiques de Cilansetron stabilisées contre la racémisation

(30) Priorität: 01.08.1997 DE 19733271; 27.03.1998 DE 19813661
(43) Veröffentlichungstag der Anmeldung: 10.02.1999
(73) Patentinhaber: Solvay Pharmaceuticals GmbH, 30173 Hannover (DE)
(72) Erfinder: Bonnacker, Ingo, 31171 Nordstemmen (DE); Köhn, Hartmut, 29223 Celle (DE); Kristen, Gerhard, 31303 Burgdorf (DE); Reichel, Christine, 30900 Wedemark (DE)
(74) Vertreter: Gosmann, Martin, Dr.

(56) Entgegenhaltungen:
- EP-A- 0 601 345
- EP-A- 0 768 309
- OHTA M ET AL.: "Novel 5HT3 Receptor Antaqgonists. III. Pharmacological Evaluations and Molecular Modeling Studies of Optically Active 4,5,6,7-Tetrahydro-1H-benzimidazole Derivatives" CHEM. PHARM. BULL., Bd. 44, Nr. 9, 1996, Seiten 1707-1716, XP000941351
- TESTA B ET AL: "RACEMATES VERSUS ENANTIOMERS IN DRUG DEVELOPMENT: DOGMATISM OR PRAGMATISM?" CHIRALITY,US,WILEY-LISS, NEW YORK, Bd. 2, 1990, Seiten 129-133, XP000653443 ISSN: 0899-0042

## Beschreibung

Die vorliegende Erfindung betrifft Cilansetron als Wirkstoff enthaltende feste und flüssige pharmazeutische Zubereitungen, welche eine zur Stabilisierung von Cilansetron gegen Racemisierung ausreichende Menge von physiologisch verträglichen wasserlöslichen sauren Zusatzstoffen enthalten. Weiterhin betrifft die Erfindung die Verwendung dieser sauren Zusatzstoffe zur Stabilisierung von Cilansetron gegen Racemisierung in pharmazeutischen Zubereitungen.

Cilansetron ist die generische Bezeichnung für das R-(-) 5,6,9,10-Tetrahydro-10-[(2-methyl-imidazol-1-yl)methyl]-4H-pyrido[3,2,1-jk]carbazol-11(8H)-on, welches aus der EP-B 0 297 651 bekannt ist. Die Verbindung kann auf an sich bekannte Weise nach den in diesem Patent angegebenen oder dazu analogen Verfahren hergestellt werden. Weiterhin ist aus der EP-A 0 768 309 ein Verfahren zur enantiomerenreinen Gewinnung von Cilansetron bekannt. Cilansetron besitzt 5 HT-antagonistische Eigenschaften und ist als pharmazeutischer Wirkstoff verwendbar. Aus der EP-B 0 601 345 ist die Verwendung von Cilansetron zur Behandlung von Krankheiten der unteren Darmwege bekannt.

Cilansetron ist eine optisch aktive Verbindung. In pharmazeutischen Zubereitungen kann Cilansetron mit der Zeit, insbesondere bei offener Lagerung, durch Umwelteinflüsse teilweise racemisieren, so daß dann in den pharmazeutischen Zubereitungen neben Cilansetron auch dessen optisches Isomer vorliegen kann. Für pharmazeutische Zubereitungen ist es jedoch wünschenswert, daß der darin enthaltene Wirkstoff in weitgehend gleichbleibender, einheitlicher Form vorliegt.

Der vorliegenden Erfindung liegt daher die Aufgabe zugrunde, Cilansetron oder dessen physiologisch verträgliche Säureadditionssalze als Wirkstoff enthaltende flüssige und feste pharmazeutische Zubereitungen bereitzustellen, worin Cilansetron gegen Racemisierung stabilisiert ist.

Gegenstand der Erfindung sind feste oder flüssige pharmazeutische Zubereitungen, enthaltend als Wirkstoff Cilansetron oder dessen physiologisch verträgliche Säureadditionssalze in üblichen therapeutisch wirksamen Mengen, dadurch gekennzeichnet, daß die Zubereitungen zusätzlich zu der zur Bildung des Säureadditionssalzes von Cilansetron nötigen Säure eine zur Stabilisierung von Cilansetron gegen Racemisierung geeignete Menge mindestens eines physiologisch verträglichen wasserlöslichen sauren Zusatzstoffes enthalten, wobei in festen Zubereitungen das molare Verhältnis wasserlöslicher Säurebestandteile zu Cilansetron in der Zubereitung zwischen 1,02:1 und 10:1 liegt und der Anteil an sauren Zusatzstoffen nicht über 50 Ges.-% der Zubereitung beträgt und wobei flüssige Zubereitungen wäßrige Lösungen darstellen, deren pH-Wert zwischen 2,5 und 4,5 liegt. Ferner ist Gegenstand der Erfindung die Verwendung dieser sauren Zusatzstoffe zur Stabilisierung von Cilansetron oder dessen Säureadditionssalzen gegen Racemisierung, insbesondere in pharmazeutischen Zubereitungen.

Erfindungsgemäß werden Cilansetron und dessen physiologisch verträgliche Säureadditionssalze gegen Racemisierung stabilisiert. Als Säureadditionssalze eignen sich die Salze von Cilansetron mit anorganischen Säuren, beispielsweise Schwefelsäure oder Halogenwasserstoffsäuren, insbesondere Chlorwasserstoffsäure, oder mit physiologisch verträglichen organischen Säuren. Vorzugsweise wird als Säureadditionssalz das Cilansetron-Hydrochlorid eingesetzt, welches in fester Form üblicherweise als Monohydrat vorliegt.

Zur Stabilisierung von Cilansetron oder dessen Säureadditionssalzen gegen Racemisierung in festen oder flüssigen pharmazeutischen Zubereitungen eignen sich als physiologisch verträgliche wasserlösliche saure Zusatzstoffe ein- oder mehrbasige organische Säuren mit 2 bis 12 Kohlenstoffatomen und einem ersten pK_{S}-Wert zwischen 1,1 und 4,8, saure Salze der mehrbasigen vorgenannten organischen Säuren und saure Salze mehrbasiger anorganischer Säuren mit einem ersten verfügbaren pK_{S}-Wert zwischen 1,5 und 7,5 sowie Gemische dieser Verbindungen. In festen pharmazeutischen Zubereitungen werden feste wasserlösliche saure Zusatzstoffe eingesetzt. In flüssigen pharmazeutischen Zubereitungen können feste oder flüssige wasserlösliche saure Zusatzstoffe verwendet werden.

Als feste saure Zusatzstoffe können beispielsweise Malonsäure, Mandelsäure, Oxalsäure, Milchsäure, Lactobionsäure, Fumarsäure, Maleinsäure, Weinsäure, Citronensäure, Ascorbinsäure oder physiologisch verträgliche saure Salze dieser Säuren verwendet werden. Auch saure Salze anorganischer Säuren, beispielsweise saure Salze von Phosphorsäure oder Schwefelsäure sowie Gemische dieser Verbindungen, vorzugsweise saure Salze von Phosphorsäure wie physiologisch verträgliche Dihydrogenphosphate, kommen in Frage. In flüssigen pharmazeutischen Zubereitungen können neben den vorstehend genannten festen sauren Zusatzstoffen auch physiologisch verträgliche flüssige organische Säuren, beispielsweise Essigsäure, verwendet werden.

Als saure Salze mehrbasiger Säuren kommen beispielsweise deren physiologisch verträgliche Alkali- oder Erdalkalimetallsalze, insbesondere deren Natrium-, Kalium- oder Calciumsalze oder deren Ammoniumsalze in Frage.

Pharmazeutische Zubereitungen von Cilansetron im Sinne der Erfindung enthalten Cilansetron in üblichen pharmazeutisch wirksamen Mengen. Beispielsweise können feste pharmazeutische Zubereitungen Cilansetron in Mengen zwischen etwa 10 mg und etwa 250 mg pro Gramm der Zubereitung enthalten, während flüssige Zubereitungen Cilansetron üblicherweise in Mengen von etwa 1 mg bis etwa 10 mg pro Milliliter der Zubereitung enthalten.

Erfindungsgemäß stabilisierte Zubereitungen enthalten vorzugsweise Säureadditionssalze des Cilansetrons, insbesondere das Cilansetron-Hydrochlorid. Es zeigt sich, daß die zur Bildung der Säureadditionssalze von Cilansetron nötige Menge an Säuren alleine üblicherweise nicht ausreicht, um Cilansetron wirkungsvoll gegen Racemisierung zu schützen. Als wasserlösliche Säurebestandteile enthalten die pharmazeutischen Zubereitungen daher die zur Bildung der Säureadditionssalze von Cilansetron nötigen Säuren sowie zusätzliche saure Zusatzstoffe. Erst durch die erfindungsgemäße Formulierung der pharmazeutischen Zubereitungen mit einem physiologisch verträglichen wasserlöslichen sauren Zusatzstoff wird eine zufriedenstellende Stabilisierung von Cilansetron gegen Racemisierung zuverlässig erreicht.

Sofern in festen pharmazeutischen Zubereitungen organische Säuren mit einem ersten pK_{S}-Wert zwischen 1,1 und 4,8 als saure Zusatzstoffe verwendet werden, sollte das Molverhältnis von wasserlöslichen Säurebestandteilen zu Cilansetron zwischen etwa 1,02:1 und etwa 5,0:1, vorzugsweise zwischen etwa 1,15:1 und etwa 3,0:1 betragen. Sofern ein Säureadditionssalz des Cilansetrons vorliegt, wird ein entsprechender Teil des Säuregehalts der Zubereitung durch den im Säureadditionssalz vorliegenden Säuregehalt geliefert. In festen pharmazeutischen Formulierungen von Säureadditionssalzen des Cilansetrons liegt daher das Molverhältnis von zugesetzten wasserlöslichen sauren Zusatzstoffen zu Cilansetron-Säureadditionssalz vorteilhaft zwischen etwa 0,02:1 und etwa 4,0:1, vorzugsweise zwischen etwa 0,15:1 und etwa 2,0:1. So wird beispielsweise Cilansetron in üblichen, das Cilansetron-Hydrochlorid-Monohydrat in einer Menge von 4,68 mg enthaltenden Tabletten von 150 mg, durch einen Gehalt an Citronensäure von zwischen etwa 0,05 mg und etwa 10,0 mg, vorzugsweise zwischen etwa 0,3 mg und etwa 4,0 mg, wirkungsvoll gegen Racemisierung stabilisiert. Die Verwendung fester organischer Säuren mit einem ersten pK_{S}-Wert zwischen 1,1 und 4,8 in festen pharmazeutischen Zubereitungen ist bevorzugt. Insbesondere kann Ascorbinsäure und/oder Citronensäure verwendet werden.

Sofern in festen pharmazeutischen Zubereitungen saure Zusatzstoffe mit einem höheren pK_{S}-Wert, beispielsweise zwischen 4,8 und 7,5, verwendet werden, sollte das Molverhältnis von wasserlöslichen Säurebestandteilen zu Cilansetron zweckmäßigerweise zwischen etwa 4:1 und etwa 10:1, vorzugsweise zwischen etwa 5:1 und etwa 8:1 betragen. Auch die Verwendung dieser schwächer sauren Zusatzstoffe in festen pharmazeutischen Zubereitungen von Cilansetron kann eine merkliche Stabilisierung des Wirkstoffes gegen Racemisierung bewirken, insbesondere bei Zutritt von Feuchtigkeit.

Sofern feste pharmazeutische Zubereitungen Säureadditionssalze von Cilansetron enthalten, ist das Cilansetron in diesen Zubereitungen umso stabiler gegen Racemisierung, je höher der Gehalt an Cilansetron-Säureadditionssalz in der betrachteten Zubereitung ist. In Abhängigkeit vom Gehalt des Cilansetron-Säureadditionssalzes in der festen Zubereitung können daher im angegebenen Mengenbereich höhere oder niedrigere Mengen an sauren Zusatzstoffen zugesetzt werden. So kann beispielsweise auch in festen Zubereitungen mit einem höheren Gehalt an Cilansetron-Säureadditionssalz, beispielsweise in Tabletten von 150 mg Gewicht mit einem Gehalt von 18, 72 mg Cilansetron-Hydrochlorid-Monohydrat, durch Zugabe eines sauren Zusatzstoffes noch eine zusätzliche Stabilisierung des Wirkstoffes gegen Racemisierung erreicht werden.

Insgesamt sollte der Anteil an sauren Zusatzstoffen 50 Gew.-% der festen Zubereitungen nicht überschreiten, um eine gute Verarbeitbarkeit der Mischungen, beispielsweise eine gute Verpreßbarkeit zu Tabletten, zu gewährleisten.

Zur Erfindung. gehören daher auch feste pharmazeutische Zubereitungen, worin das molare Verhältnis wasserlöslicher Säurebestandteile zu Cilansetron in der Zubereitung zwischen 1,02:1 und 10:1 liegt und wobei der Anteil an sauren Zusatzstoffen nicht über 50 Gew.-% der Zubereitung beträgt.

Bevorzugt sind solche erfindungsgemäße feste Zubereitungen, deren durch Auflösen in der 2500-fachen Gewichtsmenge Wasser, bezogen auf die in der Zubereitung enthaltene Menge an Cilansetron, entstandene wäßrige Lösungen oder Suspensionen pH-Werte zwischen 2,5 und 4,5, vorzugsweise zwischen 3,0 und 4,0 aufweisen. So ergeben sich beispielsweise pH-Werte von 3,8 bis 4,0, wenn man übliche Tabletten von 150 mg Gewicht und einem Cilansetron-Gehalt von 4 mg der in Tabelle 1 angegebenen erfindungsgemäßen Zusammensetzungen 1 bis 4 in 10,0 ml Wasser zerfallen läßt und nach vollständiger Lösung der wasserlöslichen Bestandteile den pH-Wert auf an sich bekannte Weise bestimmt. Dagegen ergeben sich pH-Werte von 5,0 bis 5,3, wenn man anstatt der erfindungsgemäßen Tabletten 1 bis 4 nichtstabilisierte Vergleichszusammensetzungen, beispielsweise die Tabletten 1a bis 4a in Tabelle 1, unter den gleichen Bedingungen untersucht. Zur Erfindung gehören auch solche der als bevorzugt bezeichneten vorgenannten festen pharmazeutischen Zubereitungen, welche 0,15 bis 2,0 Mol Ascorbinsäure und/oder Citronensäure auf ein Mol Cilansetron-Hydrochlorid sowie zusätzlich übliche pharmazeutische Hilfs- und/oder Trägerstoffe enthalten.

Um Cilansetron in festen pharmazeutischen Zubereitungen gegen Racemisierung zu stabilisieren, ist ein Gesamtgehalt an wasserlöslichen Säurebestandteilen zwischen etwa 5·10⁻⁵ Mol und etwa 2,5·10⁻³ Mol, vorzugsweise zwischen 6·10⁻⁵ Mol und 8·10⁻⁴ Mol, pro Gramm der festen Zubereitungen meist ausreichend. Ein höherer Gehalt an sauren Zusatzstoffen ist ebenfalls möglich, begründet aber in der Regel keinen zusätzlichen Stabilisierungseffekt mehr.

Als Beispiele fester pharmazeutischer Zubereitungen seien oral applizierbare Präparate wie Tabletten, Dragees, Kapseln, Pulver oder Granulate genannt.

Üblicherweise enthalten erfindungsgemäße feste pharmazeutische Zubereitungen noch übliche Hilfs- und/oder Trägerstoffe wie an sich bekannte Füllmittel, Bindemittel, Sprengmittel, Fließregulierungsmittel oder Trennmittel.

Als Füllmittel können Zucker wie Lactose, Zuckeraustauschstoffe wie Mannit oder Xylit, Cellulose oder Cellulosederivate wie mikrokristalline Cellulose, gegebenenfalls modifizierte Stärken wie gegebenenfalls prägelatinierte Maisstärke, oder gängige anorganische Füllmittel, beispielsweise Bentonit verwendet werden.

Als weitere Hilfsstoffe, welche beispielsweise als Bindemittel, Sprengmittel, Fließregulierungsmittel und/oder Trennmittel verwendet werden können, kommen quervernetzte Polymere von Stärkederivaten wie beispielsweise Crosscarmelose-Natrium oder Polyvinylpyrrolidonderivate wie quervernetztes Polyvinylpyrrolidon, bevorzugt Cross-PVP, kolloidales Siliciumdioxid oder langkettige amphiphile organische Verbindungen wie Stearinsäure oder Glycerinfettsäureester in Frage. Vorzugsweise sollten nur im wesentlichen neutral reagierende Hilfs- und/oder Trägerstoffe in erfindungsgemäßen festen Zubereitungen enthalten sein.

Erfindungsgemäß ist Cilansetron oder dessen Säureadditionssalz in festen pharmazeutischen Zubereitungen zusammen mit mindestens einem physiologisch verträglichen wasserlöslichen sauren Zusatzstoff und mindestens einem der oben angegebenen Hilfs- und/oder Trägerstoffe enthalten. Der Wirkstoff kann mit dem sauren Zusatzstoff und den pharmazeutischen Hilfs- und/oder Trägerstoffen in an sich bekannter Weise gemischt und formuliert werden. Zur Herstellung der festen Arzneiformen kann das Cilansetron mit den weiteren genannten Bestandteilen in üblicher Weise gemischt und naß oder trocken granuliert werden. Sofern saure Zusatzstoffe mit einem pK_{S}-Wert zwischen 4,8 und 7,5 verwendet werden, kann es vorteilhaft sein, naß zu granulieren. Das Granulat oder Pulver kann direkt in Kapseln abgefüllt oder in üblicher Weise zu Tablettenkernen verpreßt werden. Diese können gewünschtenfalls in bekannter Weise dragiert oder befilmt werden.

Bei der Herstellung erfindungsgemäßer fester Zubereitungen ist es vorteilhaft, zuerst Cilansetron oder dessen Säureadditionssalz mit nur einem Teil der Hilfs- und/oder Trägerstoffe, vorzugsweise mit etwa 5-50 Gew.-% der zur Herstellung einer festen Zubereitung insgesamt nötigen Menge an Hilfs- und/oder Trägerstoffen, und mindestens einem sauren Zusatzstoff als Vormischung auf an sich bekannte Weise zu granulieren und dieser Vormischung sodann erst die weiteren restlichen Hilfs- und/oder Trägerstoffe einzeln oder als vorgemischtes Granulat zuzugeben. Hierdurch wird eine innige Durchmischung und ein direkter Kontakt von Cilansetron mit den sauren Zusatzstoffen in den festen Zubereitungen erreicht, wodurch die Stabilisierung von Cilansetron gegen Racemisierung besonders günstig beeinflußt wird. Besonders vorteilhaft können auf diese Weise feste pharmazeutische Zubereitungen, insbesondere Tabletten, mit einem verhältnismäßig geringen Wirkstoffgehalt, beispielsweise Tabletten von 150 mg Gewicht und einem Gehalt von 2 mg Cilansetron, hergestellt werden, oder auch feste Zubereitungen, welche als saure Zusatzstoffe nur solche mit einem pK_{S}-Wert zwischen 4,8 und 7,5 enthalten.

Als flüssige Zubereitungen kommen wäßrige Lösungen von Cilansetron in Frage, welche in üblichen flüssigen Applikationsformen, beispielsweise Ampullen, abgefüllt werden können. Wirkstoff und Säurebestandteile sind in diesen flüssigen Zubereitungen naturgemäß homogen verteilt.

Üblicherweise wird zur Herstellung von flüssigen pharmazeutischen Zubereitungen ein Säureadditionssalz des Cilansetrons, bevorzugt dessen Hydrochlorid, eingesetzt. Sofern Cilansetron als Base eingesetzt wird, wird eine entsprechend höhere Menge saurer Zusatzstoffe benötigt, um die erfindungsgemäße Stabilisierung des Wirkstoffes gegen Racemisierung zu erzielen. Der pH-Wert flüssiger Zubereitungen sollte auf einen gewünschten Wert zwischen pH 2,5 und 4,5, bevorzugt zwischen pH 3,0 und pH 4,0, eingestellt werden. Insbesondere eignen sich hierzu an sich bekannte, im Bereich zwischen pH 2,5 und pH 4,5 einstellbare physiologisch verträgliche Puffersysteme wie Citratpuffer, Phosphatpuffer und/oder Acetatpuffer. Vorzugsweise kann ein Citratpuffer verwendet werden.

Zur Bildung eines geeigneten Puffersystems können die physiologisch verträglichen wasserlöslichen sauren Zusatzstoffe vorzugsweise zusammen mit einer zur Bildung eines physiologisch verträglichen Puffersystems ausreichenden Menge einer geeigneten Base eingesetzt werden. Als Basen eignen sich beispielsweise schwache Basen wie schwach basische Salze der erfindungsgemäß verwendbaren organischen Säuren. Es kann auch eine in einer zur in situ Bildung eines basischen Salzes oder eines physiologischen Puffersystems geeignete Menge einer stärkeren Base wie eines Alkalimetallhydroxyds, beispielsweise Natriumhydroxyd, verwendet werden. Sofern beispielsweise Citronensäure als saurer Zusatzstoff zu einer flüssigen pharmazeutischen Zubereitung verwendet wird, kann die Bildung eines physiologisch verträglichen Puffersystems durch Zugabe einer geeigneten Menge Natriumhydroxyd oder durch Zugabe einer geeigneten Menge Natriumcitrat erreicht werden.

In den erfindungsgemäß stabilisierten flüssigen Zubereitungen kann der Gesamtgehalt an sauren Zusatzstoffen sowie das Verhältnis von sauren Zusatzstoffen zu Cilansetron in einem relativ weiten Bereich variieren. So können die sauren Zusatzstoffe in einer Menge von etwa 2,5·10⁻⁶ Mol, bis etwa 10,0·10⁻⁵ Mol, vorzugsweise von 7,5·10⁻⁶ Mol bis 1,5·10⁻⁵ Mol pro Milliliter der Zubereitung enthalten sein. Das Verhältnis von sauren Zusatzstoffen zu Säureadditionssalz des Cilansetrons kann beispielsweise zwischen 0,15:1 und 8,0:1 betragen. Demgemäß liegt das molare Verhältnis gelöster Säurebestandteile zu Cilansetron in den flüssigen Zubereitungen dann zwischen 1,15:1 und 9,0:1. Zur Erfindung gehören auch solche flüssigen pharmazeutischen Zubereitungen, worin das molare Verhältnis gelöster Säurebestandteile zu Cilansetron zwischen 1,15:1 und 9,0:1 liegt und welche 0,3 bis 2,0 Mol Ascorbinsäure und/oder Citronensäure und/oder deren physiologisch verträgliche Salze auf ein Mol Cilansetron-Hydrochlorid sowie gegebenenfalls zusätzlich übliche pharmazeutische Hilfs- und/oder Zusatzstoffe enthalten. Aus Gründen der besseren physiologischen Verträglichkeit können flüssige Zubereitungen bevorzugt sein, worin das Verhältnis von sauren Zusatzstoffen zu Säureadditionssalz des Cilansetrons zwischen 0,3:1 und 2,0:1 beträgt.

Die flüssigen Präparate können gegebenenfalls übliche Verdünnungsmittel wie Wasser, Öle, Emulgatoren und/oder Suspensionsmittel wie Polyethylenglykole und dergleichen enthalten. Zusätzlich können gewünschtenfalls weitere Hilfs- und/oder Zusatzstoffe zugegeben werden, wie z. B. Konservierungsmittel, Geschmackskorrigenzien und dergleichen. Gewünschtenfalls können flüssige Zubereitungen vor oder nach der Abfüllung noch sterilisiert werden. Bei der Herstellung der flüssigen pharmazeutischen Zubereitungen kann es vorteilhaft sein, unter Lichtschutz zu arbeiten.

Die nachfolgenden Beispiele sollen die Erfindung näher erläutern, ohne sie in ihrem Umfang zu beschränken.

In den Beispielen wurde gereinigtes Wasser nach den Erfordernissen des Deutschen Arzneibuches (= DAB) verwendet. Der pH-Wert des verwendeten Wassers wurde vor jeder Versuchsdurchführung entsprechend den Vorschriften der "United States Pharmacopoeia" (= USP) bestimmt und betrug in allen Fällen zwischen 6,0 und 7,0. Üblicherweise wurde ein pH-Wert des verwendeten Wassers von 6,5 gemessen.

Die Bestimmung des Gehaltes an (R)- bzw. (S)- Enantiomer des Wirkstoffes erfolgte jeweils durch High Performance Liquid Chromatography (= HLPC) an chiralem Säulenmaterial (Chiradex, Firma Merck).

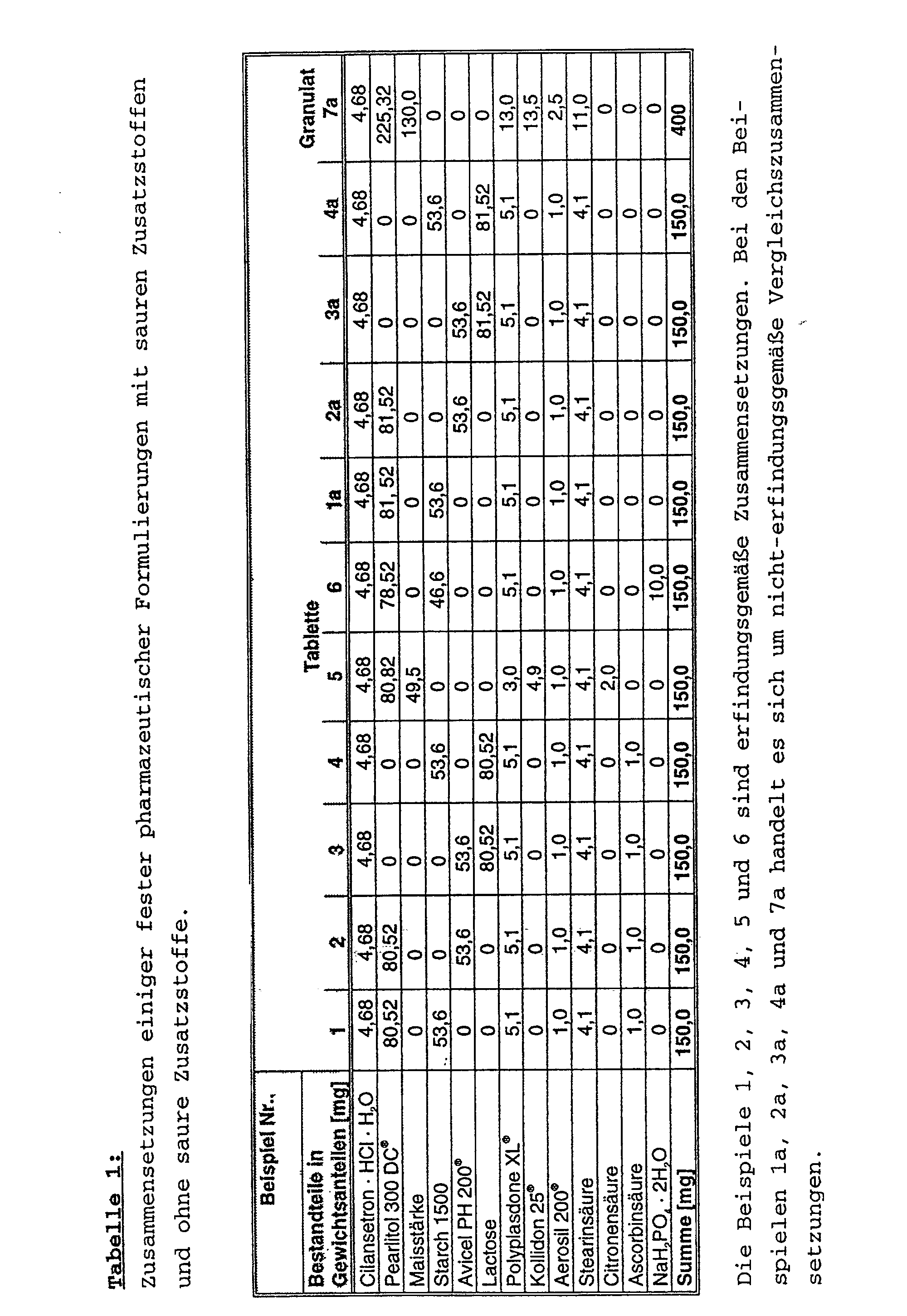

### Beispiele 1-4 und 6 und Vergleichszusammensetzungen 1a - 4a:

### Herstellung von Tabletten mit und ohne saurer Komponente durch Direkttablettierung

Die in Tabelle 1 angegebenen Tablettenformulierungen 1 bis 4, 1a bis 4a und 6 wurden durch Direkttablettierung hergestellt. Hierzu wurde der Wirkstoff oder eine den Wirkstoff und den sauren Zusatzstoff enthaltende Vormischung mit den angegebenen Hilfsstoffen aus der Gruppe Mannit (Pearlitol 300 DC®, Firma Roquette), feingepulverte Lactose, Maisstärke, prägelatinierte Maisstärke (Starch 1500®, Firma Colorcon) und/oder mikrokristalline Cellulose (Avicel PH 201®, Firma FMC) vermischt. Dann wurde hochdisperses Siliciumdioxid (Aerosil 200®, Firma Degussa), Stearinsäure und quervernetztes Polyvinylpyrrolidon (Polyplasdone XL®, Firma GAF Chemicals) zugemischt und die entstandenen Mischungen wurden auf einer Rundläuferpresse zu Tabletten von je 150 mg Gewicht verpreßt, so daß jede Tablette 4 mg des reinen Wirkstoffes enthielt.

### Beispiele 5 und 7a:

### Herstellung von Tabletten und Kapseln mit saurem Zusatzstoff und ohne sauren Zusatzstoff durch Granulierung

Die in Tabelle 1 angegebene Tablettenformulierung 5 und das Granulat 7a wurden durch ein Granulationsverfahren hergestellt. Hierzu wurde der Wirkstoff mit dem Mannit und der Maisstärke in einem Mischer vermischt und im Falle des Beispiels 5 wurde die entstandene Mischung mit der Citronensäure, gelöst in der notwendigen Menge einer 20%igen Lösung von Polyvinylpyrrolidon (Kollidon 25®, Firma BASF) in entmineralisiertem Wasser, durchfeuchtet. Falls erforderlich, wurde in beiden Beispielen, 5 und 7a, entmineralisiertes Wasser hinzugefügt. Das feuchte Gemisch wurde in einem hochtourigen Mischer (Fa. Diosna) granuliert, und das entstandene Rohgranulat wurde bei 40 °C auf Horden getrocknet und durch ein Sieb gegeben. Anschließend wurde das hochdisperse Siliciumdioxid, die Stearinsäure und das Polyvinylpyrrolidon beigemischt. Das fertige Granulat wurde dann je nach Formulierung entweder in Portionen von 400 mg mittels automatischer Kapselmaschine in Hartgelatinekapseln der Größe 0 abgefüllt, so daß jede Kapsel 4 mg des reinen Wirkstoffes enthielt (Granulat 7a) oder auf einer Rundläuferpresse zu Tabletten von je 150 mg Gewicht verpreßt, so daß jede Tablette 4 mg Cilansetron enthielt (Tablette 5).

### Beispiel 8:

### Herstellung flüssiger pharmazeutischer Zubereitungen (Ampullen) mit saurem Zusatzstoff

Es wurde eine flüssige Zubereitung von Cilansetron mit Citratpuffer als saurem Zusatzstoff der Zusammensetzung:

| | |
|---|---|
| Cilansetron · HCl · H₂O | 234 mg |
| Citronensäure-Monohydrat | 60 mg |
| NaCl | 900 mg |
| NaOH | 5 mg |
| entmineralisiertes Wasser | 99,296 g |

hergestellt. Der pH-Wert der Lösung betrug etwa 3,6. Hierzu wurde der Wirkstoff und die Hilfsstoffe unter Lichtschutz in dem Wasser gelöst, wobei die Lösung ständig mit Stickstoff begast wurde. Die Lösung wurde dann durch Membranfilter der Porenweite 0,2 µm filtriert und mittels einer automatischen Ampullenabfüllanlage in Portionen von 2 ml in Ampullen der Größe 2 ml abgefüllt, so daß jede Ampulle 4 mg Cilansetron-Base enthielt.

### Beispiel 9:

Vergleich der pH-Werte in wäßrigen Lösungen oder Suspensionen verschiedener fester pharmazeutischer Zubereitungen mit sauren Zusatzstoffen und ohne saure Zusatzstoffe.

Die erfindungsgemäßen festen pharmazeutischen Formulierungen 1-6 sowie die nicht-erfindungsgemäßen Vergleichszusammensetzungen 1a-4a und 7a, welche vorstehend in Tabelle 1 angegeben sind, wurden bei Raumtemperatur jeweils in 10,0 ml Wasser (pH = 6,5) gegeben. Die Formulierung des Beispiels 7a wurde unter den gleichen Bedingungen in 25,0 ml Wasser gegeben. Nach Zerfallen der Zubereitungen und vollständiger Auflösung der wasserlöslichen Bestandteile wurden die pH-Werte der entstandenen wäßrigen Lösungen oder Suspensionen jeweils mit einer Glaselektrode gemessen. Die erhaltenen pH-Werte sind in der nachfolgenden Tabelle 2 angegeben.

**Tabelle 2:**

| | | | | | | | | | | | |
|---|---|---|---|---|---|---|---|---|---|---|---|
| **Feste pharmazeutische Formulierung Nr.** | 1 | 2 | 3 | 4 | 5 | 6 | 1a | 2a | 3a | 4a | 7a |
| **pH-Wert** | 4,0 | 4,0 | 3,9 | 3,9 | 3,3 | 4,8 | 5,3 | 5,0 | 5,2 | 5,1 | 5,2 |

Eine der Wirkstoffmenge der obigen Formulierungen entsprechende Menge an Cilansetron-Hydrochlorid-Monohydrat von 4,68 mg wurde unter den vorstehend angegebenen Bedingungen in 10,0 ml Wasser gelöst. Der pH-Wert der entstandenen Lösung wurde zu 5,45 bestimmt.

### Stabilitätsuntersuchungen I:

### Vergleich der Racemisierungsgeschwindigkeiten von Cilansetron in Tabletten mit und ohne saurer Komponente

Die in Tabelle 3 aufgeführten Tablettenformulierungen mit (betrifft Tabletten 1 bis 4) und ohne (betrifft Tabletten 1a bis 4a) Zusatz von saurem Zusatzstoff wurden einem Lagertest unterworfen. Nach 4 Wochen wurde jeweils die Zunahme des Gehaltes an durch Racemisierung entstandenem S-(+)-Isomer von Cilansetron in den einzelnen Formulierungen bestimmt. Die Ergebnisse des Lagertests sind in Tabelle 3 dokumentiert.

**Tabelle 3:**

| Zunahme des Gehaltes an S-(+)-Isomer von Cilansetron in Tabletten mit saurem Zusatzstoff und ohne sauren Zusatzstoff nach 4-wöchiger Lagerung | | | | | | | | | | |
|---|---|---|---|---|---|---|---|---|---|---|
| **Lagerbedingung** | **relative Feuchtigkeit [%]** | **Zunahme des Gehaltes an S-(+)-Enantiomer in %** | | | | | | | | |
| | | **Tablette** | | | | | | | | |
| | | **1** | **2** | **3** | **4** | **6** | **1a** | **2a** | **3a** | **4a** |
| 30 °C geschlossen | 60 | 0,10 | 0,10 | 0,10 | 0,20 | -- | 0,20 | 0,20 | 0,20 | 0,30 |
| 30 °C offen | 60 | 0,10 | 0,10 | 0,40 | 0,20 | -- | 1,70 | 0,20 | 0,20 | 0,50 |
| 40 °C offen | 75 | 0,40 | 0,40 | 0,40 | 0,40 | 0,10 | 1,70 | 0,70 | 0,70 | 1,70 |
| 50 °C geschlossen | unbestimmt | 1,60 | 1,10 | 1,60 | 1,70 | -- | 0,70 | 3,50 | 2,30 | 2,20 |

Wie die in Tabelle 3 angegebenen Meßwerte zeigen, lassen sich bereits nach 4-wöchiger Lagerung deutliche Unterschiede im Gehalt an durch Racemisierung entstandenem S-(+)-Enantiomer feststellen, insbesondere bei offener Lagerung, d. h. bei Zutritt von Luft und Feuchtigkeit und gegebenenfalls erhöhter Temperatur. In den erfindungsgemäß stabilisierten Formulierungen ist dabei die Zunahme des Gehaltes an S-(+)-Enantiomer signifikant niedriger als in den unstabilisierten Vergleichsformulierungen.

### Stabilitätsuntersuchungen II:

### Racemisierungsgeschwindigkeit von Cilansetron in flüssigen Zubereitungen mit verschiedenen pH-Werten

Es wurde eine wäßrige, Citrat-gepufferte Stammlösung von Cilansetron der Zusammensetzung:

| | |
|---|---|
| Cilansetron · HCl · H₂O | 6,684 g |
| Citronensäure-Monohydrat | 30,2 g |
| NaCl | 9,0 g |
| NaOH | 11,5 g |
| 1N HCl | 1566,0 g |

hergestellt. Aus dieser Stammlösung wurden durch Zugabe der jeweils notwendigen Menge an 1N HCl Probenlösungen mit pH-Werten von 2,9; 3,3; 3,6; 3,8 und 4,0 hergestellt. Die Probenlösungen wurden einem Lagertest bei zwei verschiedenen Temperaturen (26 °C und 41 °C) unterworfen und der Gehalt an durch Racemisierung des Wirkstoffes entstandenem S-(+)-Enantiomer wurde nach Zeitintervallen von 8 Wochen (Lagertemperatur 26 °C) bzw. nach 12 Wochen und nach 12 Monaten (Lagertemperatur 41 °C) bestimmt. Die Ergebnisse dieses Lagertests sind in Tabelle 4 aufgetragen.

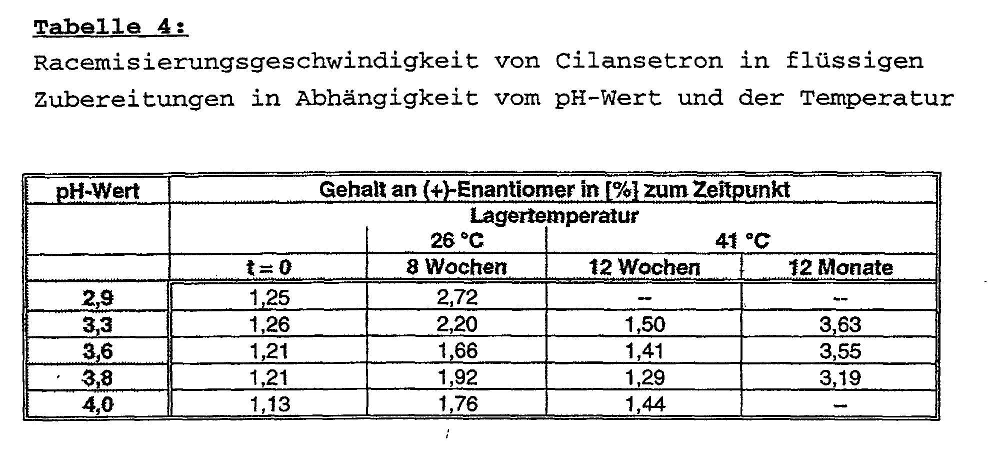

Aus den in Tabelle 4 angegebenen Meßwerten ist zu ersehen, daß Cilansetron im untersuchten pH-Bereich am wirkungsvollsten zwischen pH 3,6 und pH 4,0 gegen Racemisierung geschützt wird.

### Stabilitätsuntersuchungen III:

### Einfluß des pH-Wertes und der Temperatur auf die Racemisierungsgeschwindigkeit von Cilansetron in flüssigen Zubereitungen

Cilansetron-Hydrochlorid-Monohydrat wurde in einer Konzentration von 1 % in 0,065 molarem Phosphatpuffer gelöst. Aus dieser Stammlösung wurden durch Zugabe der notwendigen Menge an 1/15 molarer wäßriger Natronlauge Probenlösungen mit pH-Werten von 2, 3, 4, 5 und 6 hergestellt. Die einzelnen Probenlösungen wurden bei 61 °C jeweils 1, 7, 14 und 28 Tage lang gelagert und die Geschwindigkeitkonstanten der Racemisierung von Cilansetron wurden unter Annahme einer Kinetik 1. Ordnung auf bekannte Weise ermittelt. Die Ergebnisse dieses Tests sind in Tabelle 5 aufgetragen.

**Tabelle 5:**

| Geschwindigkeitskonstanten der Racemisierung von Cilansetron in flüssigen Zubereitungen bei unterschiedlichen pH-Werten und 61 °C | | | | | |
|---|---|---|---|---|---|
| **pH-Wert** | 2 | 3 | 4 | 5 | 6 |
| **k*10**^{**-3**}**[1/d]** | 8,86 | 3,00 | 2,21 | 7,18 | 21,05 |

Aus den in Tabelle 5 aufgetragenen Meßwerten ist zu ersehen, daß die Racemisierungsgeschwindigkeit von Cilansetron bei pH-Werten größer als pH 2 und kleiner als pH 5, vorzugsweise zwischen pH 3 und pH 4, signifikant geringer ist, als bei anderen pH-Werten.

### Stabilitätsuntersuchungen IV:

### Lagerstabilität von Cilansetron in flüssigen Zubereitungen

Auf die in Beispiel 8 angegebene Weise wurde eine Citrat-gepufferte flüssige Zubereitung von Cilansetron mit einem Wirkstoffgehalt von 2 mg/ml hergestellt und in zwei verschiedenen Ampullengrößen zu 2 ml und 4 ml abgefüllt. Der pH-Wert der Lösungen betrug jeweils 3,7. Die Ampullen wurden einem Lagertest unterworfen. Nach 6 Monaten und nach 24 Monaten wurde jeweils der Gehalt an S-(+)-Enantiomer in den Ampullen bestimmt. Die Meßwerte sind in Tabelle 6 aufgetragen.

**Tabelle 6:**

| Nachweis der Lagerstabilität von Cilansetron in erfindungsgemäß stabilisierten flüssigen pharmazeutischen Formulierungen | | | | | | |
|---|---|---|---|---|---|---|
| **Produkt** | **pH-Wert** | **Gehalt an (+)-Enantiomer in [%]** | | | | |
| | | **t = 0** | **6 Monate** | | **24 Monate** | |
| | | | **40 °C** | **k 10**^{**-3**} **[1/d]** | **25 °C** | **k 10**^{**-5**} **[1/d]** |
| **Ampullen, 4 mg/2 ml** | 3,7 | 1,6 | 4,0 | 1,37 | 3,7 | 3,0 |
| **Ampullen 8 mg/4 ml** | 3,7 | 1,5 | 3,8 | 1,31 | 3,9 | 3,4 |

Aus den berechneten Geschwindigkeitskonstanten k ergibt sich, daß bei einer durchschnittlichen Lagertemperatur von 25 °C der Anteil an S-(+)-Enantiomer an der Wirkstoffmenge in den Ampullen etwa 3 Jahre lang den Wert von 5 % nicht übersteigen wird.

## Patentansprüche

1. Feste oder flüssige pharmazeutische Zubereitungen, enthaltend als Wirkstoff Cilansetron oder dessen physiologisch verträgliche Säureadditionssalze in üblichen therapeutisch wirksamen Mengen, **dadurch gekennzeichnet, daß** die Zubereitungen zusätzlich zu der zur Bildung des Säureadditionssalzes von Cilansetron nötigen Säure eine zur Stabilisierung von Cilansetron gegen Racemisierung geeignete Menge mindestens eines physiologisch verträglichen wasserlöslichen sauren Zusatzstoffes enthalten, wobei in festen Zubereitungen das molare Verhältnis wasserlöslicher Säurebestandteile zu Cilansetron in der Zubereitung zwischen 1,02:1 und 10:1 liegt und der Anteil an sauren Zusatzstoffen nicht über 50 Gew.-% der Zubereitung beträgt und wobei flüssige Zubereitungen wäßrige Lösungen darstellen, deren pH-Wert zwischen 2,5 und 4,5 liegt.

2. Pharmazeutische Zubereitungen gemäß Anspruch 1, worin der wasserlösliche saure Zusatzstoff ausgewählt ist aus der Gruppe bestehend aus ein- oder mehrbasigen organischen Säuren mit 2 bis 12 Kohlenstoffatomen und einem ersten pK_{S}-Wert zwischen 1,1 und 4,8, sauren Salzen der mehrbasigen vorgenannten organischen Säuren und sauren Salzen physiologisch verträglicher mehrbasiger Mineralsäuren mit einem ersten verfügbaren pK_{S}-Wert zwischen 1,5 und 7,5.

3. Pharmazeutische Zubereitungen gemäß Anspruch 2, welche als saure Zusatzstoffe Ascorbinsäure, Citronensäure, Fumarsäure, Lactobionsäure, Maleinsäure, Malonsäure, Mandelsäure, Milchsäure, Oxalsäure, Weinsäure und/oder saure Salze der mehrbasigen vorgenannten organischen Säuren und/oder physiologisch verträgliche Dihydrogenphosphate und/ oder Hydrogensulfate oder, falls die Zubereitung flüssig ist, auch physiologisch verträgliche flüssige organische Säuren enthalten.

4. Pharmazeutische Zubereitungen gemäß einem der vorstehenden Ansprüche, worin als Säureadditionssalz des Cilansetrons das Cilansetron-Hydrochlorid enthalten ist.

5. Feste phamazeutische Zubereitungen gemäß einem der vorstehenden Ansprüche, worin als saure Zusatzstoffe feste ein- oder mehrbasige organische Säuren mit 2 bis 12 Kohlenstoffatomen und einem ersten pK_{S}-Wert zwischen 1,1 und 4,8 enthalten sind.

6. Feste pharmazeutische Zubereitungen gemäß Anspruch 5, worin das molare Verhältnis wasserlöslicher Säurebestandteile zu Cilansetron in der Zubereitung zwischen 1,02:1 und 5,0:1 liegt, wobei der Anteil an sauren Zusatzstoffen nicht über 50 Gew.-% der Zubereitung beträgt.

7. Feste pharmazeutische Zubereitungen gemäß einem der vorstehenden Ansprüche, deren durch Auflösen der festen Zubereitungen in der jeweils 2500-fachen Gewichtsmenge Wasser, bezogen auf die in den Zubereitungen enthaltene Menge an Cilansetron, entstandene wäßrige Lösungen oder Suspensionen, pH-Werte zwischen 2,5 und 4,5 aufweisen.

8. Feste pharmazeutische Zubereitungen gemäß Anspruch 7, welche 0,15 bis 2,0 Mol Ascorbinsäure und/oder Citronensäure auf ein Mol Cilansetron-Hydrochlorid sowie zusätzlich übliche pharmazeutische Hilfs- und/oder Trägerstoffe enthalten.

9. Flüssige pharmazeutische Zubereitungen gemäß Anspruch 1, welche ein auf einen pH-Wert zwischen 2,5 und 4,5 eingestelltes physiologisch verträgliches Puffersystem enthalten.

10. Flüssige pharmazeutische Zubereitungen gemäß Anspruch 9, welche mindestens ein Puffersystem, ausgewählt aus einem Citratpuffer, einem Phosphatpuffer und einem Acetatpuffer, enthalten.

11. Flüssige pharmazeutische Zubereitungen gemäß Anspruch 1, worin das molare Verhältnis gelöster Säurebestandteile zu Cilansetron in den Zubereitungen zwischen 1,15:1 und 9,0:1 liegt.

12. Flüssige pharmazeutische Zubereitungen gemäß Anspruch 11, welche 0,3 bis 2,0 Mol Ascorbinsäure und/oder Citronensäure und/oder deren physiologisch verträgliche Salze auf ein Mol Cilansetron-Hydrochlorid sowie gegebenenfalls zusätzlich übliche pharmazeutische Hilfs- und/oder Zusatzstoffe enthalten.

13. Verwendung von physiologisch verträglichen wasserlöslichen sauren Zusatzstoffen, ausgewählt aus organischen ein- oder mehrbasigen Säuren mit 2 bis 12 Kohlenstoffatomen und einem ersten pK_{S}-Wert zwischen 1,1 und 4,8, sauren Salzen der mehrbasigen vorgenannten organischen Säuren und sauren Salzen physiologisch verträglicher mehrbasiger Mineralsäuren mit einem ersten verfügbaren pK_{S}-Wert zwischen 1,5 und 7,5 zur Stabilisierung von Cilansetron gegen Racemisierung in pharmazeutischen Zubereitungen nach Anspruch 2.

14. Verfahren zur Herstellung fester pharmazeutischer Zubereitungen nach Anspruch 2, enthaltend als Wirkstoff Cilansetron oder dessen physiologisch verträgliche Säureadditionssalze und zusätzlich mindestens einen physiologisch verträglichen festen wasserlöslichen sauren Zusatzstoff, welcher ausgewählt ist aus der Gruppe bestehend aus ein- oder mehrbasigen organischen Säuren mit 2 bis 12 Kohlenstoffatomen und einem ersten pK_{S}-Wert zwischen 1,1 und 4,8, sauren Salzen der mehrbasigen vorgenannten organischen Säuren und sauren Salzen mehrbasiger Mineralsäuren mit einem ersten verfügbaren pKₛ-Wert zwischen 1,5 und 7,5, **dadurch gekennzeichnet, daß** man zuerst Cilansetron oder ein Säureadditionssalz von Cilansetron mit mindestens einem sauren Zusatzstoff und mit 5-50 Gew.-% der zur Herstellung der festen Zubereitungen insgesamt nötigen Menge an Hilfs- und/oder Trägerstoffen zu einer Vormischung vermischt und die erhaltene Vormischung gewünschtenfalls granuliert, dann die restlichen Hilfs- und/oder Trägerstoffe hinzugibt und gewünschtenfalls das auf diese Weise erhaltene Granulat oder Pulver zu Tabletten verpreßt oder in übliche Dosierungsformen abfüllt.

## Claims

1. Liquid or solid pharmaceutical preparations, containing as active substance cilansetron or its physiologically compatible acid addition salts in conventional therapeutically effective quantities, **characterised in that** the preparations, in addition to the acid necessary for the formation of the acid addition salt of cilansetron, contain a quantity, suitable for the stabilising of cilansetron against racemisation, of at least one physiologically compatible water-soluble acid additive, wherein in solid preparations the molar ratio of water-soluble acid components to cilansetron in the preparation lies between 1.02:1 and 10:1, and the proportion of acid additives does not amount to over 50 % by weight of the preparation, and wherein liquid preparations represent aqueous solutions which have pH values between 2.5 and 4.5.

2. Pharmaceutical preparations according to Claim 1, wherein the water-soluble acid additive is selected from the group consisting of monobasic or multibasic organic acids with 2 to 12 carbon atoms and a first pKₛ value between 1.1 and 4.8, acid salts of the multibasic above-mentioned organic acids and acid salts of physiologically compatible multibasic mineral acids with a first available pKₛ value between 1.5 and 7.5.

3. Pharmaceutical preparations according to Claim 2, which contain as acid additives ascorbic acid, citric acid, fumaric acid, lactobionic acid, maleic acid, malonic acid, mandelic acid, lactic acid, oxalic acid, tartaric acid and/or acid salts of the multibasic above-mentioned organic acids and/or physiologically compatible dihydrogen phosphates and/or hydrogen sulphates or, if the preparation is liquid, also contain physiologically compatible liquid organic acids.

4. Pharmaceutical preparations according to one of the above claims, wherein cilansetron hydrochloride is contained as acid addition salt of cilansetron.

5. Solid pharmaceutical preparations according to one of the above claims, wherein solid monobasic or multibasic organic acids with 2 to 12 carbon atoms and a first pKₛ value between 1.1 and 4.8 are contained as acid additives.

6. Solid pharmaceutical preparations according to Claim 5, wherein the molar ratio of water-soluble acid components to cilansetron in the preparation lies between 1.02:1 and 5.0:1, the proportion of acid additives not amounting to over 50% by weight of the preparation.

7. Solid pharmaceutical preparations according to one of the preceding claims, the aqueous solutions or suspensions of which, produced by dissolving the solid preparations in 2500 times the weight quantity of water in each case, in relation to the quantity of cilansetron contained in the preparations, have pH values between 2.5 and 4.5.

8. Solid pharmaceutical preparations according to Claim 7, which contain 0.15 to 2.0 mole ascorbic acid and/or citric acid to one mole cilansetron hydrochloride and also additionally usual pharmaceutical adjuvants and/or excipients.

9. Liquid pharmaceutical preparations according to Claim 1, which contain a physiologically compatible buffer system set at a pH value between 2.5 and 4.5.

10. Liquid pharmaceutical preparations according to Claim 9, which contain at least one buffer system, selected from a citrate buffer, a phosphate buffer and an acetate buffer.

11. Liquid pharmaceutical preparations according to Claim 11, wherein the molar ratio of dissolved acid components to cilansetron in the preparations lies between 1.15:1 and 9.0:1.

12. Liquid pharmaceutical preparations according to Claim 13, which contain 0.3 to 2.0 mole ascorbic acid and/or citric acid and/or their physiologically compatible salts to one mole cilansetron hydrochloride and also optionally in addition usual pharmaceutical adjuvants and/or additives.

13. The use of physiologically compatible water-soluble acid additives, selected from organic monobasic or multibasic acids with 2 to 12 carbon atoms and a first pKₛ value between 1.1 and 4.8, acid salts of the multibasic above-mentioned organic acids and acid salts of physiologically compatible multibasic mineral acids with a first available pKₛ value between 1.5 and 7.5 for the stabilising of cilansetron against racemisation in pharmaceutical preparations according to Claim 2.

14. A method for the production of solid pharmaceutical preparations according to Claim 2, containing as active substance cilansetron or its physiologically compatible acid addition salts and in addition at least one physiologically compatible solid water-soluble acid additive, which is selected from the group consisting of monobasic or multibasic organic acids with 2 to 12 carbon atoms and a first pKₛ value between 1.1 and 4.8, acid salts of the multibasic above-mentioned organic acids and acid salts of multibasic mineral acids with a first available pKₛ value between 1.5 and 7.5, **characterised in that** firstly cilansetron or an acid addition salt of cilansetron is mixed with at least one acid additive and with 5-50% by weight of the total quantity of adjuvants and/or excipients necessary for the production of the solid preparations to form a pre-mixture and the resulting pre-mixture is granulated if desired, then the remaining adjuvants and/or excipients are added and if desired the granules or powder obtained in this way are/is compressed into tablets or filled into conventional dosaging forms.

## Revendications

1. Préparations pharmaceutiques solides ou liquides contenant comme substance active du cilansetron ou ses sels d'addition d'acides physiologiquement acceptables, en quantités thérapeutiquement efficaces habituelles, **caractérisées en ce que** ces préparations, outre l'acide nécessaire à la formation du sel d'addition d'acide de cilansetron, contiennent une quantité d'au moins un additif acide soluble dans l'eau, physiologiquement acceptable, appropriée pour stabiliser le cilansetron contre une racémisation, le rapport molaire dans les préparations solides des composants acides solubles dans l'eau au cilansetron étant dans la préparation compris entre 1,02:1 et 10:1, la fraction d'additifs acides ne devant pas dépasser 50 % en poids de la préparation, les préparations liquides se présentant sous forme de solutions aqueuses au pH compris entre 2,5 et 4,5.

2. Préparations pharmaceutiques selon la revendication 1, dans lesquelles l'additif acide soluble dans l'eau est choisi dans le groupe constitué par les acides organiques mono- ou polybasiques comportant de 2 à 12 atomes de carbone et présentant un premier pK_{A} compris entre 1,1 et 4,8, les sels acides des acides organiques polybasiques mentionnés ci-dessus et les sels acides d'acides inorganiques polybasiques physiologiquement acceptables ayant un premier pK_{A} disponible compris entre 1,5 et 7,5.

3. Préparations pharmaceutiques selon la revendication 2, qui contiennent comme additifs acides l'acide ascorbique, l'acide citrique, l'acide fumarique, l'acide lactobionique, l'acide maléique, l'acide malonique, l'acide mandélique, l'acide lactique, l'acide oxalique, l'acide tartrique et/ou les sels acides des acides organiques polybasiques mentionnés ci-dessus et/ou des dihydrogénophosphates physiologiquement acceptables et/ou des sulfates d'hydrogène ou encore, si la préparation est liquide, des acides organiques liquides physiologiquement acceptables.

4. Préparations pharmaceutiques selon l'une des revendications précédentes, qui contiennent comme sel d'addition d'acide de cilansetron le chlorhydrate de cilansetron.

5. Préparations pharmaceutiques solides selon l'une des revendications précédentes, qui contiennent en tant qu'additifs acides, des acides organiques mono- ou polybasiques comportant 2 à 12 atomes de carbone et présentant un premier pK_{A} compris entre 1,1 et 4,8.

6. Préparations pharmaceutiques solides selon la revendication 5, dans lesquelles le rapport molaire des constituants acides solubles dans l'eau au cilansetron dans la préparation se situe entre 1,02:1 et 5:1, la proportion des additifs acides ne dépassant pas 50 % en poids de la préparation.

7. Préparations pharmaceutiques solides selon l'une des revendications précédentes, dont les solutions ou suspensions aqueuses obtenues par dissolution des préparations solides dans une quantité pondérale respectivement 2 500 fois plus importante d'eau, par rapport à la quantité de cilansetron contenue dans les préparations, présentent des pH compris entre 2,5 et 4,5.

8. Préparations pharmaceutiques solides selon la revendication 7, qui contiennent 0,15 à 2,0 moles d'acide ascorbique et/ou d'acide citrique pour une mole de chlorhydrate de cilansetron ainsi que des auxiliaires et/ou véhicules pharmaceutiques supplémentaires habituels.

9. Préparations pharmaceutiques liquides selon la revendication 1, qui contiennent un système tampon physiologiquement acceptable dont le pH est ajusté entre 2,5 et 4,5.

10. Préparations pharmaceutiques liquides selon la revendication 9, qui contiennent au moins un système tampon choisi parmi un tampon de citrate, un tampon de phosphate et un tampon d'acétate.

11. Préparations pharmaceutiques liquides selon la revendication 1, dans lesquelles le rapport molaire des constituants acides dissous au cilansetron dans les préparations se situe entre 1,15:1 1 et 9,0:1.

12. Préparations pharmaceutiques liquides selon la revendication 11, qui contiennent 0,3 à 2,0 moles d'acide ascorbique et/ou d'acide citrique et/ou de leurs sels physiologiquement acceptables pour une mole de chlorhydrate de cilansetron et éventuellement des auxiliaires et/ou additifs pharmaceutiques supplémentaires habituels.

13. Utilisation d'additifs acides solubles dans l'eau physiologiquement acceptables, choisis parmi les acides organiques mono- ou polybasiques comportant 2 à 12 atomes de carbone et ayant un premier pK_{A} compris entre 1,1 et 4,8, les sels acides des acides organiques polybasiques mentionnés ci-dessus et les sels acides d'acides inorganiques polybasiques physiologiquement compatibles ayant un premier pK_{A} disponible entre 1,5 et 7,5 pour la stabilisation du cilansetron contre une racémisation dans les préparations pharmaceutiques selon la revendication 2.

14. Procédé d'élaboration de préparations pharmaceutiques solides selon la revendication 2, contenant comme substance active du cilansetron ou ses sels d'addition d'acides physiologiquement acceptables et, en outre, au moins un additif acide soluble dans l'eau solide et physiologiquement acceptable, choisi dans le groupe constitué par les acides organiques mono- ou polybasiques comportant 2 à 12 atomes de carbone et ayant un premier pK_{A} compris entre 1,1 et 4,8, les sels acides des acides organiques polybasiques mentionnés ci-avant et les sels acides d'acides inorganiques polybasiques ayant un premier pK_{A} disponible compris entre 1,5 et 7,5, **caractérisé en ce qu'**on mélange tout d'abord le cilansetron, ou un sel d'addition d'acide de cilansetron avec au moins un additif acide et avec 5 à 50 % en poids de la quantité globalement nécessaire d'auxiliaires et/ou de véhicules pour élaborer les préparations solides, pour obtenir un prémélange, et **en ce que** si on le désire, on granule le prémélange obtenu, puis **en ce qu'**on ajoute le reste d'auxiliaires et/ou de véhicules et, si on le désire, **en ce qu'**on comprime le produit granulé et/ou la poudre obtenu(e) de cette manière en comprimés ou **en ce qu'**on le ou la conditionne dans des formes posologiques habituelles.
